# EUROPEAN PATENT APPLICATION

(11) **EP 3 975 200 A1**
(43) Date of publication of application: **30.03.2022**
(21) Application number: 20198490.3
(22) Date of filing: 25.09.2020
(51) Int. Cl.: G16H 40/20, G16H 10/60

(54) **REDUCTION OF HEALTHCARE VARIATION**

(71) Applicant: HITACHI, LTD., Chiyoda-ku Tokyo 100-8280 (JP)
(72) Inventor: GHAFOURI, Mustafa, London, Greater London EC2Y 5AJ (GB)
(74) Representative: Mewburn Ellis LLP

(57) **Abstract**

A computer-implemented method for identifying unwarranted healthcare variation in a healthcare system. The method comprises: (a) acquiring healthcare process data, the healthcare process data describing events occurring in relation to a plurality of patients in the healthcare system; (b) analysing the healthcare process data to identify an actual clinical pathway taken by each patient through the healthcare system, and to determine variations in those pathways; and (c) providing the determined variations to a clinical process visualizer, the clinical process visualizer displaying the determined variations for interpretation.

## Description

### Field of the Invention

The present disclosure relates to a computer implemented method for reducing unwarranted healthcare variation in a healthcare system.

### Background

Healthcare variation (both clinical and operational variation) is a major problem in modern healthcare systems, and is defined as the overuse, underuse, different use, and waste of healthcare practices and services, resulting in variation in patient outcomes and/or key performance metrics. In essence, healthcare variation revolves around the different healthcare pathways that patients with similar medical conditions and symptoms take through a healthcare system, and why patients with similar conditions and symptoms take different pathways through a healthcare system. It also relates to assessing the impact that this has on patient outcomes and key performance metrics. Patients with the same, or similar, medical conditions should ideally receive the same treatment. Variation analytics looks at why this is not the case, and tries to reduce the variation. Elimination of variation is critical not only to ensuring that patients receive the safest, highest-quality care, but it is also integral to reducing the overall cost of healthcare for individual patients, as well as healthcare providers.

Healthcare variation includes warranted variation or unwarranted variation. Warranted variation is justifiable and acceptable. This is because some variation observed in healthcare either occurs by random unpredictable chance or arises because each patient is different and needs to be cared for as an individual with specific symptoms, characteristics, needs, personal circumstances, and values.

Variation in healthcare which is not justifiable is called unwarranted variation. It is variation that can only be explained by differences in healthcare system performance, rather than due to patient or doctor preferences, individual patient condition, or random chance. Unwarranted clinical variation can have a negative impact on patients' access to services and health outcomes, including mortality rates. For example, there is unwarranted variation between healthcare providers for crude mortality rates in the UK. This shows a range from 3.4% crude mortality rates for some healthcare providers, to 13.6% for others. Some of these differences can be explained by justifiable and warranted variation (such as having different types and groups of patients). However much of the variation in crude mortality rates between healthcare providers can only be explained by differences in quality of healthcare being provided, and therefore is unwarranted variation.

Clinical variation exists for multiple reasons, including the supply of patient-sensitive care, where multiple options exist as well as patient or doctor's preferences, and the availability of services. In many cases, it is not possible to be definitive about all of the reasons for unwarranted variation because the delivery of healthcare is complex and it is not possible to control all of the variables involved.

Nonetheless, it is desirable to better track and monitor healthcare variation, so as to allow unwarranted variation to be reduced. Previous methods attempting this are too slow as to provide meaningful impacts on the healthcare service, and require substantial amounts of human resource.

### Summary

Accordingly, in a first aspect, the present disclosure provides a computer-implemented method for identifying unwarranted healthcare variation in a healthcare system, the method comprising:
(a) acquiring healthcare process data, the healthcare process data describing events occurring in relation to a plurality of patients in the healthcare system;
(b) analysing the healthcare process data to identify an actual clinical pathway taken by each patient through the healthcare system, to determine variations in those pathways; and
(c) providing the determined variations to a clinical process visualizer, the clinical process visualizer displaying the determined variations for interpretation.

Such a method allows for unwarranted healthcare variation in a healthcare system to be reduced, and so the method has a technical effect, the reduction of unwarranted variation, on a process, the provision of healthcare, carried on outside of the computer.

The method may have any one or, to the extent that they are compatible, any combination of the following optional features.

The method may include varying a healthcare system workflow to be experienced by one or more of the patients based on the determined variations.

In step (b), variations may be determined between the actual clinical pathways for respective patients. In step (b), variations may be determined between the actual clinical pathway for each patient and a reference clinical pathway.

The healthcare process data may describe events occurring in relation to a plurality of patients in the healthcare system, and the plurality of patients may be selected based on an expected clinical pathway.

The healthcare process data may describe events occurring in relation to a plurality of patients in the healthcare system, and the plurality of patients may be selected based on priority rankings of the patients.

The method may include a step, performed before step (a), of receiving one or more key performance metrics associated with the healthcare system, and the data acquired in step (a) may at least partially describe the key performance metrics. For example, the one or more key performance metrics may be selected from the list including: a cost incurred until discharge, for both a standard clinical pathway and a clinical pathway including unwarranted variation; a cost incurred until discharge for a best practice clinical pathway; a duration of hospital stay; a patient satisfaction score; a patient waiting time; an accident rate; a number of clinical mistakes; a bed turnover rate; and a number of staff active for a period of time.

The healthcare process data may be acquired in the form of process cubes, which contain an event log structure at a plurality of levels of granularity. The process cubes may be online analytical processing (OLAP) cubes, i.e. a multi-dimensional dataset. The process cubes may integrate data from a plurality of data sources. Advantageously, the use of process cubes allows for analysis to be performed at an arbitrary level of granularity.

Acquiring the healthcare process data may include identifying missing or erroneous event data, and providing or correcting the missing or erroneous event data by imputation before the healthcare process data is analysed. The imputation of the missing or erroneous data may use real time location systems which track one or more physical objects associated with a patient. For example, the real time location system may track the patient by tracking a bracelet or locator attached to the patient or their bed, it may also track a medical device, a medical consumable, or other clinical items. The imputation of the missing or erroneous data may use data from a plurality of sources, linked by an event occurring in relation to a given patient. Advantageously, the use of a real time location system significantly improves the accuracy with which missing or erroneous event data is corrected by imputation.

Analysing the healthcare process data may include applying a conformance checking algorithm, which is configured to determine an individual patient's conformance to a reference clinical pathway. The reference clinical pathway may be derived by pre-analysing the healthcare process data to determine a clinically desired pathway.

Identifying the actual clinical pathway taken by each patient may be identified by use of either a hierarchical process mining method, or a declarative process mining method.

The method may comprise repeating steps (a) - (c).

The method may further include a step of deriving, from the determined variations, an improvement to the healthcare system to reduce unwarranted healthcare variation. The method may include a step of implementing the improvement to the healthcare system.

In a second aspect, the present disclosure provides a computer system for identifying unwarranted healthcare variation in a healthcare system, the system comprising one or more processors and memory, the memory containing machine executable instructions which, when run on the one or more processors, causes the one or more processors to:
(a) acquire healthcare process data, the healthcare process data describing events occurring in relation to a plurality of patients in the healthcare system;
(b) analyze the healthcare process data to identify an actual clinical pathway taken by each patient through the healthcare system, and to determine variations in those pathways; and
(c) provide the determine variations to a clinical process visualizer, the clinical process visualizer displaying the determined variations for interpretation.

The memory may contain machine executable instructions which, when run on the one or more processors, cause the one or more processors to perform the computer-implemented method of the first aspect including any one, or any combination insofar as they are compatible, of the optional features set out with reference thereto.

Further aspects of the present disclosure provide: a computer program comprising code which, when run on a computer, causes the computer to perform the method of the first aspect; a computer readable medium storing a computer program comprising code which, when run on a computer, causes the computer to perform the method of the first aspect; and a computer system programmed to perform the method of the first aspect.

### Brief Description of the Drawings

Embodiments of the present disclosure will now be described by way of example with reference to the accompanying drawings in which:
Figure 1A shows a process cube and Figure 1B shows an example of the contents of a process cube;
Figure 2 shows an example process map for sepsis management in a hospital created from event logs;
Figure 3 shows an example of a macro analysis process;
Figure 4 shows an example of information displayed on a clinical process visualizer;
Figure 5 is a schematic of a system;
Figure 6 is a schematic showing further detail of the process mining system 502 of Figure 5; and
Figure 7 is a flow diagram.

### Detailed Description and Further Optional Features

Aspects and embodiments of the present disclosure will now be discussed with reference to the accompanying figures. Further aspects and embodiments will be apparent to those skilled in the art.

### Data engineering and integration of disparate data sources

Before variation analytics can be conducted, it is desirous to first process captured data in a useful format so that variation analysis can be optimised (including using process mining and the use of a clinical process visualizer, which is discussed in detail later). It is also important that the data can be segmented (this can be referred to as 'slicing and dicing') into the appropriate segments for analysis. That is the focus of this section.

Data for process mining is, in this example, in the format of an event log, which is a data format appropriate for event data. An example of an event log is shown below:

**Table 1**

| **Patient ID** | **Event ID** | **Activity** | **Start Date** | **Department** | **Surgery** |
|---|---|---|---|---|---|
| 1045 | 12 | Admission to hospital | 3 January 2017 09:47 | Cardiovascular surgery | Coronary artery bypass |
| 1045 | 13 | Surgery started | 4 January 2017 08:00 | Cardiovascular surgery | Coronary artery bypass |
| 1045 | 14 | Transfer to ICU | 4 January 2017 14:53 | Cardiovascular surgery | Coronary artery bypass |
| 1045 | 15 | Surgery finished | 4 January 2017 14:55 | Cardiovascular surgery | Coronary artery bypass |
| 1045 | 16 | Surgery started | 5 January 2017 06:15 | Cardiovascular surgery | Postoperative exploration |
| 1045 | 17 | Surgery finished | 5 January 2017 07:50 | Cardiovascular surgery | Postoperative exploration |
| 1045 | 18 | Transfer to service | 13 January 2017 11:20 | Cardiovascular surgery | Postoperative exploration |
| 1045 | 19 | Discharged | 20 January 2017 11:49 | Cardiovascular surgery | Postoperative exploration |

There are four columns in a typical event log: a case or patient ID column, an event ID column, an activity or activity ID column, and a time column (this can include a start time and end time for a given event). Additional columns can be included, describing attributes of the event (such as department and surgery, as shown in the example above).

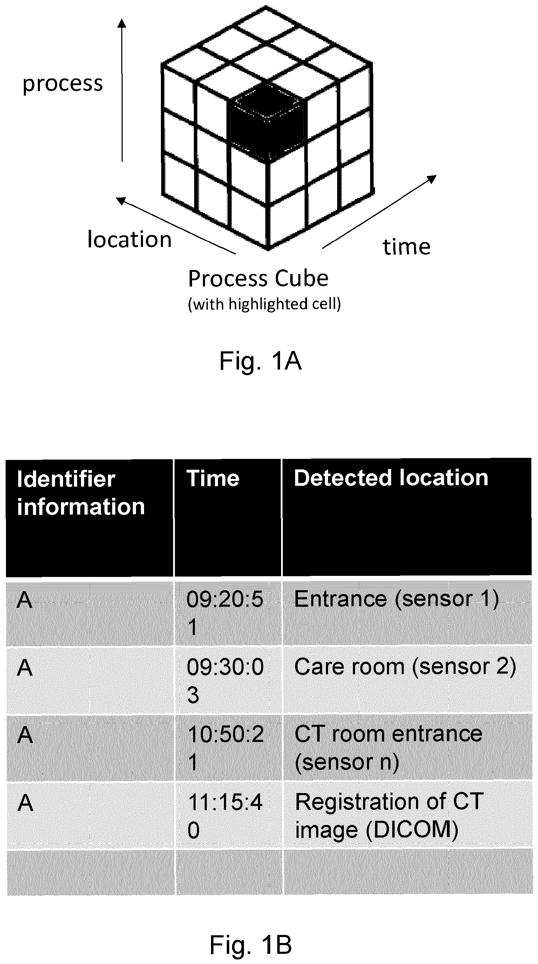

According to the present disclosure, a version of online analytical processing (OLAP), which is referred to as an event-based OLAP or process cube is utilised, in order to be able to segment the relevant extract of data in a structured event log format. The process cube integrates multiple data sources whilst retaining the event log format. A process cube therefore allows for the segmentation of data (e.g. by key-performance indicator outcome, case or event attribute, department, diagnosis, etc.) to provide segment event logs from the process cubes utilising the integrated data from multiple disparate data sources. An example of a process cube is shown in Figure 1A, and can be considered a three dimensional array: a process array; a location array; and a time array. Example contents of a process cube is shown in Figure 1B. A cell, as highlighted in Figure 1A, corresponds to a particular process occurring at a particular location at a particular time.

The present disclosure addresses the integration of data from multiple, disparate sources, which might be missing or have inaccurate contents. Missing or inaccurate event data means forming incomplete event logs, which can hamper the reduction of healthcare variation as complete healthcare pathways cannot always be identified or analysed.

### Inaccurate data

A common reason for inaccurate data is that the time at which a specific event actually occurs is different to that input into a computer system for recording. Particularly for a healthcare environment, an activity may occur for a group of patients, such as when administering medications to a group of patients. The activity may be logged after all medications have been administered to all patients, rather than after a specific medication has been administered to an individual patient. Accordingly, it would appear on the computer system that all medicine administration occurred simultaneously for all patients.

The present disclosure addresses this by utilising real-time locating system data (RTLS). RTLS improves data quality, and increases data accuracy when it is inputted to the computer system even if it is inputted after the event actually occurs. Suggestions are made to the user inputting the event of the accurate time of when the event actually occurred based on the RTLS data. Referring to the example above, if a user inputs data on medicine administration after the medicine has actually been administered, recommendations of a time data input for the medicine administration for each patient based on when the clinician was closest to that specific patient based on the RTLS data would be provided.

A further method of improving data accuracy is the automated triggering of data input. The clinician can notify the computer system that they will be conducting a task, in advance, and the system then inputs the data as the event occurs based on the RTLS data. Alternatively, the system can automatically trigger event data input utilising either preprogramed rules or predictive algorithms with RTLS data. For example, to attain the accurate time that a patient was discharged from hospital, or transferred between wards or departments, the RTLS system can automatically trigger an event when the patient leaves hospital grounds, or a designated area. Alternatively, the patient could place their wristband, with the relevant sensor or sensing system, into a box or designated area, which would automatically trigger the event that the patient was being discharged from the hospital.

### Missing data

The present disclosure also handles missing data in event logs, through methods of process imputation. A common cause of missing event data is that data is not input for events that occur without a digital trace. For example, when a clinical performs a clinical task, such as wrapping a bandage on a patients' wound, there may only be documentation in the patient's paper records and no digital records of the event. The present disclosure addresses missing data through different methods of imputation. One method of imputation would be to utilise RTLS data, including assets RTLS data. For example, if a sensor for a new bandage is next to the sensor for a patient, it can be inferred that an event where the bandage is being used for that patient has taken place.

Another method for imputation utilises an understanding of common pathways, and relationships between events. For example, when a patient has blood samples taken, although corresponding data may not be directly inputted into the computer system it is possible to infer from the subsequent laboratory investigation that this event occurred and utilise RTLS data to infer the time of the event. It is also possible to impute events by inferring information from a combination of RTLS data and an understanding of the location and/or pathway of the patient. Again, for example, if a patient spends an amount of time in an x-ray room, and other data shows a digital x-ray being performed, it is possible to impute that the patient was having an x-ray at that time. Further, it is possible to impute events based on the integration of disparate data sources into the Process Cube discussed previously. For example, by integrating data from an ECG machine and administration data, it can be imputed that the patient was having an ECG examination at a specific time.

### Levels of granularity

A further issues with integration of disparate healthcare data sources is the different levels of granularity with which that data is collected. For example, one data source may use days, whilst another integrated source may use hours and minutes. This can be alleviated by use of RTLS data (in the manner discussed above) in order to obtain more accurate time stamps or events and so integrate data sources that have different levels of granularity.

### Process mining

The present disclosure utilises process mining to understand patient pathways and identify core aspects of variation in patient healthcare pathways. Process mining is a set of techniques, algorithms, and attributed software that can perform process analysis using event data in the format of an event log as discussed above. Process mining techniques can be used to build process maps from event logs, showing core routes and pathways that a patient takes based on their event data with no human modelling required.

Further, a technique called conformance checking can be utilised to show patients following different pathways in a process map in near real time, and check the conformance of the patient to different pathways. This can be performed using historical data or on a live system.

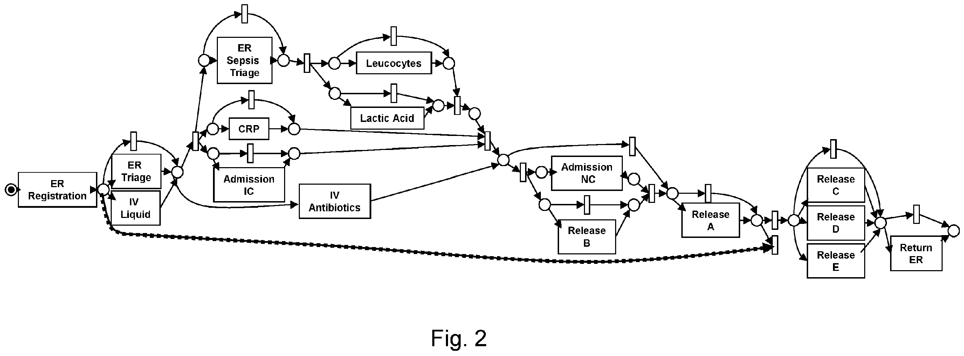

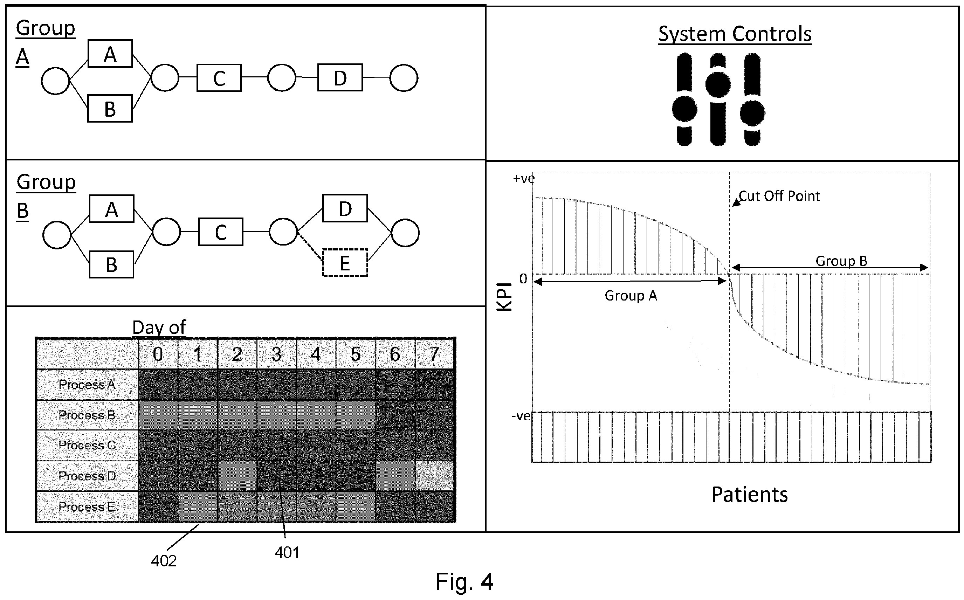

Figure 2 shows a simple process map for sepsis management in a hospital created from event logs. An animation of a patient's conformance to this pathway, as the patient moves through the pathway, can be visualised in near real time, checking conformance of the patient's pathway to this pathway and identifying patients that deviate from the pathway. In addition to the representation shown in the upper left panel of Figure 4 (discussed below), the process cubes representing the pathways would also show a difference, in that process cube for Group B would contain a further element (e.g. a dot).

### Identification of a segment of patients for variation analytics via macro-analysis

As discussed above, it is useful to be able to segment a process cube, as otherwise conducting process mining on an entire healthcare system (such as a whole hospital or Trust) would be complicated and computationally expensive. Rather, the present disclosure allows a focus on a particular segment of a given healthcare system, such as a segment of patients which may represent a healthcare pathway.

There are many method for identifying segments of patients for analysis. One method is to have experienced staff members (e.g. healthcare managers, senior clinicians, etc.) select a segment of patients or pathway on which to conduct variation analysis. A choice of areas of healthcare delivery can be made in which, according to their expert opinion, a reduction in unwarranted variation is needed. This can be based, for example, on key performance indicator (KPI) reports and management meetings, or National Institute for Health and Care Excellence (NICE) guidelines and clinical experience.

### Macro Analysis

More objective segmentation can be conducted via quantitative analysis. One form of quantitative analysis, which is used to select which segment of patients to focus variation analytics, on is called macro analysis. A macro analysis compares different groups of patients, wards, departments, healthcare pathways, etc. to identify which of these has the highest priority for performing variation analytics on. A macro analysis can consider key performance metrics in relation to their ability to identify where the maximum potential for performance improvement lies in relation to reducing unwarranted variation. The macro analysis can segment patients (e.g. into different wards, departments, disease groups, healthcare pathways, etc.) and analyse these segments by different key performance metrics, incorporating the number of patients in each segment.

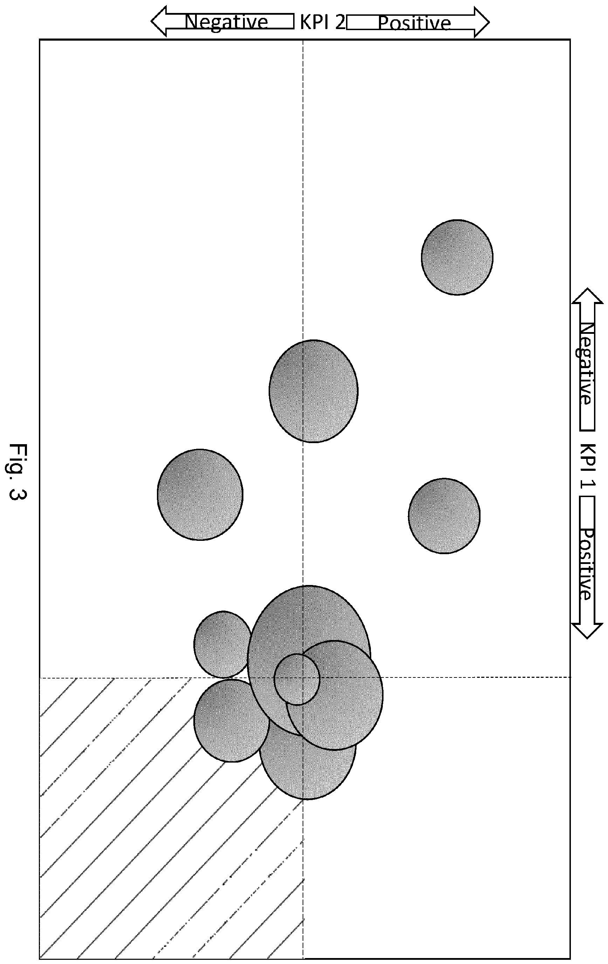

An example of a macro analysis is shown in Figure 3, in which two KPIs are considered. For example, one KPI may be length of stay (LOS) on the X axis (an important KPI for hospitals that is increased by higher levels of unwarranted variation), and profit and loss may be the other KPI shown on the Y axis. The size of the circles represents the number of patients in a segment, for example the number of inpatients in a year for a given department. The macro analysis can then be split into four quadrants: a top right quadrant, a top left quadrant, a bottom left quadrant, and a bottom right quadrant. The top left quadrant, in this example, showcases wards and departments with a low LOS and a high profit, whilst the bottom right quadrant showcases wards and departments with a high LOS and low profit. It is therefore wards and departments in the bottom right quadrant which should be prioritised for variation analytics. The specific department (i.e. segment) can be selected by a member of staff based on experience or the choice can be automated by selected e.g. the segment with the largest number of patients. In this sense, the macro analysis for choosing a segment to perform variation analysis can have staff involvement or can be fully automated. Other quantitative and digital methods can be used to identify which segment of patients to perform variation on.

### Identifying patient pathways

Once a segment of patients has been selected for variation analytics, the next step is to map the core pathways that patients in this selected segment take throughout the healthcare system. A pathway is defined as a specific ordering of work activities across time and space, with a beginning and an end, and defined inputs and outputs (e.g. a structure for action of patient activities for a specific patient). There are different aspects of the patient pathway which needs to be identified and analysed. This includes the desired pathway (what should be happening), as well as the actual pathways patients commonly take (what is actually happening), and variations in the common pathways patients take (the variation in what is happening), and how does this impact key outcomes (the impact of variation on key performance metrics).

### Identifying desired healthcare pathways

Desired pathways are often developed manually as guidelines for healthcare works to follow, to guide them in delivery of healthcare services. Pathways can be developed at different levels of healthcare delivery (e.g. national, regional, hospital, community practice, etc.) and utilise evidenced-based medicine as understood by healthcare experts to understand what the best actions for patients are. Common sources of healthcare guidelines in the UK include the NICE, NHS England, and other clinical rules repositories. Although guidelines can offer details on a desired pathway at a clinical or operational level, they need to be applied at local level taking into account local constraints, goals, and requirements (e.g. at the level of an NHS Trust or GP Partner). Local guidelines represent the interpretation of local NHS staff of NICE or NHS guidelines, and are subject to interpretation bias and the working culture of the local environment. Guidelines can be imported or downloaded into systems, according to the present disclosure, in a digital format from the relevant source (e.g. from NHS England, nice.org.uk, etc.). Guidelines can be downloaded in a format or notation which is machine readable, and digitally interactable e.g. a decision tree, business process model and notation (BPMN), Petri-nets, Workflow nets etc. The present disclosure allows a user to manually download the appropriate guideline for the segment of patients that they are focusing on. Systems according to the present disclosure can also automatically download the most appropriate pathway(s) based on the selected segment of patients. For example, for a segment consisting of patients diagnosed with colon cancer, the system can automatically identify and download relevant guidelines for the management of patients with colon cancer from any clinical rules repository or local database, in a machine-readable format. These guidelines represent pathways for patients to follow.

### Identifying actual healthcare pathways followed

So as to identify and understand variation, as well as understand the impact of variation on key performance metrics, it is helpful to understand the actual healthcare pathways that patients take. Whilst guidelines represent the ideal pathway, the real pathway that patients take through a healthcare system will often be different, resulting in variation. To understand variation, it is important to understand how patients follow healthcare pathways in real life.

One method for doing this is process discovery, where process mining algorithms analyse event log data to produce one or more process maps which represent one or more core pathways. Process mining builds data driven models of patient pathways, with no models being built manually or by users. This overcomes human limitations in reconstructing complex processes, and so models of healthcare pathways built via process mining are objective and represent a more accurate depiction of real-life hospital pathways.

One challenge addressed by the present disclosure is the high levels of variability and variation in individual patient pathways. As patients with the same symptoms or illness can follow a wide range of different pathways, mapping healthcare pathways can be complex and result in a model which is uninterpretable and therefore unusable for effective variation analysis. A further challenge addressed by the present disclosure is that, due to the turbulent nature of hospitals and other healthcare environments, patient care plans (representations of patient pathways for specific patients) are continuously updated and changed in response to outcomes from patient interventions, as well as other criteria-based progressions e.g. whether a patient progresses in the pathway to have medication, or whether their blood test result for a specific disease is positive or negative. This dynamic nature of pathways means that they continuously change as events occur.

The present disclosure utilises multiple techniques for addressing these challenges in process discovery of patient pathways. The techniques focus on aggregating processes to a higher level of abstraction and representing processes as a compact set of rules. Whilst this means losing some aspects of the granular details of a patient pathway, the pathway becomes more amenable to variation analytics. Hierarchical process mining is one technique used by the present disclosure. This technique develops a higher level of granularity for the model in iterative cycles. In hierarchical process mining, a complex model is initially built, and after each cycle a new less complex model is built with mode aggregation. The cycle stops when a threshold is reached for the overall level of complexity of the model (this threshold for the level of complexity can be chosen beforehand or chosen via an algorithm).

Another technique used by the present disclosure is declarative process mining. In declarative process mining, rules and constraints on the final process model outcome from process discovery are declared in advance. These rules and constraints ensure that the final process discovery model is practical and fit for purpose in variation analytics. Examples of rules and constraints that can be declared include having a declarative inventory of possible activities, criteria-based progressions, time-frame restrictions, etc.

These techniques are automated and can automatically select the level of granularity and detail of the final model. An alternative would be receiving an input from a user as to the level of granularity and detail for the pathways. This could be implemented through use of a slider which gives the user multiple options for the level of detail.

### Identifying variation in patient pathways and how this impacts patient outcomes and key performance metrics

### Identifying groups of patients

The present disclosure assesses the impact of variation on key performance metrics and patient outcomes by looking at different groups of patients. The present disclosure utilises clinical process visualizer (CPV) software, to facilitate quick identification of variation in clinical activities between two or more groups by first selecting a key performance metric or patient outcome to focus on, choosing a cut-off point (or multiple points when splitting into more than two groups), and dividing the patients into groups based on cut-off points. Variation can then be identified between the two or more groups.

For example, if looking at cancer waiting times, an important key performance metric is whether a patient begins treatment for their cancer within 62 days of being referred to a secondary health service by a community doctor (e.g. a GP). The present disclosure analyses variation by splitting patients into a "good group" of patients who had their treatment started before 62 days, and a "bad group" of patients who had not begun treatment within 62 days. The present disclosure then analyses and identifies the differences in variation between these two groups. This is shown schematically in Figure 4, which is an example of a clinical process visualizer.

The system, or a user, can identify the key performance metric or patient outcome on which to perform variation analysis. This can be based on, for example, clinical experience, guidelines, and/or policy. Building on the example of cancer waiting times, the key performance metric of starting treatment within 62 days of referral is a UK government policy aimed at improving cancer services in the UK and would be identified by the system or a clinical specializing in cancer as an appropriate cut-off point. Systems according to the disclosure automate the selection of key performance metrics, number of cut-off points, and value of cut-off points. This is achieved by using the key performance metrics, as well as key cut-off figures from the desired pathway. For example, if a macro-analysis according to the disclosure highlights cancer waiting times as an area to reduce variation, the system identifies the desired pathway by downloading guidelines (as discussed previously) and in those guidelines, algorithms are used to identify key performance metrics which are relevant to the pathway (e.g. time between referral by community doctor and start of treatment), and key cut-off figures for this key performance metric (e.g. 62 days). The system then uses the identified key performance metric and cut off point to separate a segment of patients into groups based on these criteria.

As well as using cut-off points of patient outcomes or key performance metrics to identify groups of patients, groups of patients can also be identified through inputs by users into the system, or through the use of algorithms which analyse reference texts or the desired pathway.

### Automatically identifying variation in process maps by comparing groups

Once the different groups of patients are identified, process mining and CPV software can be used for variation analytics. Using process discovery on each of the groups can identify differences in pathways between the groups, as well as differences in the pathways between the desired and actual pathway. Identifying variation in pathways between groups facilitates the understanding of how variation impacts patient outcomes or key performance metrics. Identifying variation between the desired pathways, and actual pathways, showcases and highlights additional and/or missing activity or processes in the actual pathway that may need to be changed to achieve the desired pathway. Because of the specific techniques used to address the challenges of high variability in process discovery, the differences identified between the different process discovery models can be identified at a useful level of granularity.

Differences in process maps can be showcased and utilised in different ways, whether comparing different groups or when comparing differences between the desired and actual pathway. One method according to the present disclosure is to highlight the differences on the process map. This is shown in Figure 4, an example of a CPV, where two process maps are illustrated: one for patient group A, and one for patient group B. As shown in Figure 4, the process map for group B highlights with a dashed line an additional process in the pathway which is often taken by patients in group B but not group A.

The comparisons between two different groups can be improved or adjusted in multiple different ways to improve performance. Analysis can be adjusted for: age; sex; gender; morbidity levels; propensity scores; etc. to allow for a more fair and accurate analysis. Other methods of data and statistical transformations can also be used to achieve the same goal of improving accuracy, fairness, or identifying different aspects of differences between groups. For example, one aspect of variation in pathways between groups may be that one group has re-work in the pathway (e.g. time spend fixing effects, or repeating tasks and processes when not required). Data and statistical transformations may be used to highlight differences in re-work between the pathways. Additionally, metadata or additional attributes of the event data can be used to gain further insight into the pathway variation identified e.g. using additional event attributes on the ward or doctor to which the patient has been assigned.

This method of identifying variation in healthcare pathways can identify multiple different types of variation including distinctive and divergent paths. Activities and processes that are missing between groups can be identified, as well as additional activities or processes between groups. Variation in the order or schedule of activities can also be identified. Differences in the frequency of activities or process, the method in which activities or processes are carried out can also be identified. Different pathways, and their attributes, can be identified for different types of patients or any other segmentation of patients (e.g. the different doctors that attend to patients or wards that the patients are attributed to).

As well as showcasing variation and differences in pathways to users, the system allows the automatic detection of differences and variations in patient pathways.

### Clinical process visualizer (CPV)

The present disclosure utilises a clinical process visualizer, with process mining as discussed above, for use in tackling healthcare pathway variation. The CPV facilitates the visualisation of key events in relative time of patient pathways. After splitting patients into groups based on a cut-off point in patient outcomes or key performance metrics, the CPV can use an algorithm to create a visualisation of a time series for all activities and processes involved in that patient care, with coding (e.g. colour coding) to indicate their impact on the patient outcomes or key performance metrics. By doing so, a CPV allows the identification of variation that impacts patient outcomes or key performance metrics. An example of a CPV tool is shown in Figure 4.

A CPV tool has two key visualisations: (i) a patent outcome or key performance metric, used to split patients at a particular cut-off point (see right hand side of Figure 4); and (ii) a visualisation of all activities as time series data (see left hand side, lower panel, of Figure 4). The time series data is ordered by day in the pathway, starting at 0, on the X axis. On the Y axis is a list of all activities or processes that could be potentially involved in patient care, including operational and clinical activities and processes. The middle section is made up of a composition of squares, with each square representing an activity or process on a particular day of the pathway. Each square has an associated coding, e.g. colour coding, with the coding of the square showing whether an activity or process performed on a day or stage of a pathway has occurred more for one group of patients than another group of patients. An uncoded or uncoloured square indicates that an activity has not occurred on a particular day of the pathway. In the example in Figure 4, a blue square 401 indicates that this activity was more likely to occur for a patient in the cost saving group, whilst a red square 402 indicates that an activity was more likely to have occurred for the overspending group on a particular day of the pathway.

A CPV therefore allows the identification of differences and variations in activities or processes in the pathway which are likely to have contributed to the grouping of patients. In the example of Figure 4, the CPV identifies which variation of activities in the pathways was likely to have influenced the patients being in the cost saving group or the overspending group. Indeed, in combination with the other methods discussed herein, a CPV can be used to identify variation in processes and activities between a desired pathway and an actual pathway.

The present disclosure utilises a number of algorithms in conjunction with a CPV. One example orders all activities and processes by the impact across time that the activity has on the grouping of the patient outcome or key performance metric. This facilitates the identification of activities or processes which have the largest impact on the patient outcome or key performance metrics. In the example shown in Table 2 below, the algorithm indicated that the variation in the prescription of the drugs Tamoxifen Citrate and Letrozole had the biggest influence on patient outcome or key performance metric (not shown in the table, the patient outcome was measured as length of stay in hospital). As well as allowing clearer and faster analysis, the algorithm also allows the automation of variation analytics in the system such that activities having the biggest impact on patient outcomes or key performance groups can be automatically identified.

**Table 2**

| Act ID | Description | Sum | Ave | PValue of Ftest | Ftest | Pvalue of Ttest | Significant difference | DTW |
|---|---|---|---|---|---|---|---|---|
| 20 | Tamoxifen Citrate | 9.8E+02 | 6.1 E+01 | 1.3E-08 | Incongruous variances | 1.7E-06 | TRUE | 2.7E+02 |
| 23 | Letrozole | -4.3E+02 | -2.7E+01 | 0.0E+00 | Incongruous variances | 8.7E-03 | TRUE | 1.7E+02 |
| 32 | Morphine | 6.0E+02 | 3.7E+01 | 2.5E-08 | Incongruous variances | 3.4E-07 | TRUE | 1.6E+02 |
| 21 | Raloxifene Hydrochlorid e | -1.4E+02 | -8.9E+00 | 5.9E-08 | Incongruous variances | 1.6E-01 | FALSE | 9.1 E+01 |
| 22 | Anastrozole | -1.0E+02 | -6.3E+00 | 4.9E-14 | Incongruous variances | 1.0E-01 | FALSE | 6.1 E+01 |
| 24 | Exemestane | -6.6E+01 | -4.1 E+00 | 1.2E-08 | Incongruous variances | 1.5E-01 | FALSE | 4.3E+01 |
| 105 | Body mass index 18.5 - 24.9 | 4.3E+01 | 2.7E+00 | 1.7E-09 | Incongruous variances | 4.1 E-01 | FALSE | 4.3E+01 |

### Focused CPV analysis on distinctive and divergent paths identified by Process Mining

The pathways identified and compared from process mining can be used for further analysis by the CPV. The CPV can be used to focus on a subset of patients, or processes and activities, that been identified as displaying variation in their pathways through Process Mining. In particular, this can be used to focus on patients that follow divergent and distinctive paths as identified by comparing process maps, which have been discovered by process mining. For example, if by using process mining a difference of additional path is discovered (e.g. taking anti-thrombin medication), further CPV-based analysis can be conducted comparing patients that have the additional path to those that do not.

There are several advantages to comparing CPV and process mining for this analysis of variation over using CPV or process mining alone. One advantage is that a more granular detail is derived on the cause and root-cause analysis of pathway variation. This can help derive underlying aspects of variation, and complex interactions of processes and activities that result in pathway variation. Additionally, this may help in differentiating warranted and unwarranted variation.

### Use of process mining to more accurately calculate the activities or processes with the most variation

As mentioned previously, the disclosure provides an algorithm for highlighting the activities or processes with the highest levels of variation impacting patient outcomes or key performance metrics. This algorithm takes into account the time series data for each process or activity across the whole time period of the pathway. Accuracy of scoring such activities or processes can be improved by adjusting the algorithm values based on the time in the pathway where it is possible to perform a given activity or process. The information on when in the healthcare pathway an activity or process can be performed can be obtained from process mining of process maps. For example, in the pathway shown in Figure 2, the unmodified algorithm would take into account all time across the entire pathway when calculating the process or activity with the most impact on patient outcome or key performance metric. The modified algorithm would use all possible time that the activity could be performed, in order to calculate the impact of the variations. As an example, for blood tests (e.g. Leucocytes, Lactic Acid, c-reactive protein) this would be the time period after emergency room (ER) triage and before admission for normal care (NC).

The combination of process mining and CPV can provide more accurate analysis of the areas of variation when combined than each method as used on its own. One method of doing this is to showcase the correlation where both methods agree there is variation. Any activity or process which is highlighted by both methods as likely showcasing larger aspects of variation is likely to showcase unwarranted variation. This allows for the more accurate monitoring of both the degree to which there is variation in an activity or process, and whether this variation is warranted or unwarranted.

When using both process mining and CPV, the two methods combined can provide supplementary information on variation. When initially identifying variation in the pathway using process mining, CPV can then be used to identify the timing of this variation in the pathway. This is because CPV identifies additional information on which day or time along the pathway the variation is occurring. Alternatively, when conducting CPV analysis to identify variation, process mining can be used to develop further insight into this variation. Process mining can be performed on a group of patients with the activity or process that has variation and compare this to a group of patients that do not have the activity or process that showcases variation. This allows further insight into the pathways which lead to the area of variation, and the impact of the variation on subsequent pathways. As well as allowing root cause analysis of variation identified by CPV, this can also highlight unwarranted and warranted variation to clinicians.

### Identifying unwarranted variation

Several methods have been discussed, in which the combination of CPV and process mining allows insight into understanding whether a variation is unwarranted or warranted. These includes detecting activities or processes with the most amount of variation (which are likely to be unwarranted variation), as well as identifying this variation more accurately using process mining on variations identified by CPV, so as to identify different pathways associated with variation and performing CPV analysis on divergent paths to understand the root cause analysis of variation, and comparing variation between desired and actual pathways.

The methods are able to identify unwarranted variation in three ways:
1) Unwarranted variation is likely to have the largest variational impact on patient outcomes or key performance metrics, and so identification of variations with the largest variational impact can be made;
2) Root cause analysis of variation, this can identify if the variation is associated with a patient choice or systemic choice of healthcare services; and
3) By comparing actual pathways to desired pathways, unwarranted variation is identified. This is because desired pathways take into account warranted variation (e.g. patient choice). This is true also when making a comparison between actual pathways and desired pathways which takes into account data from other healthcare providers, and so will showcase the warranted variation seen in other healthcare provider's services.

### Utilising identified variation to improve patient outcomes and/or key performance metrics

Once variation has been identified, this is utilised to improve patient outcomes and key performance metrics. Whilst this can be performed manually by hospital staff and change management initiatives, the present disclosure also provides several methods of using the insight developed of variation to automatically initiate change and reduce variation.

### Conformance checking for Operational Monitoring

One aspect of process mining is conformance checking. Using conformance checking during process mining allows the monitoring of identified healthcare variation and pathway conformance checking in near real time. For example, conformance checking allows near-real time monitoring of patients that follow the desired pathway, pathways associated with unwarranted variation, or patients that follow divergent and distinctive paths. By monitoring patients that follow paths likely to lead to increased unwarranted variation, resulting in reduced patient outcomes or key performance metrics, automated actions can be initiated to prevent patients following paths that in unwarranted variation. If a patient follows a path that leads to unwarranted variation, it is then possible to perform: actions including near-real time alerts and recommendations to staff, automatic updates to the hospital system automated actions, and automatic scheduling of activities including re-ordering the scheduling of activities.

Alerts can be sent to staff when patients are following a path that is likely to result in unwarranted variation, so that appropriate actions can be taken to reduce the changes of patients following this path. Recommendations can suggest that appropriate actions are taken to ensure that patients do not follow a pathway which leads to unwarranted variation but instead follow activities and processes on the desired path.

Automatic updates can be send to the hospital information system to enable activities and processes that prevent patients following paths that lead to unwarranted variation. This could include updating stock and inventory (including medications) with the required items for activities and processes that are required to reduce unwarranted variation; updating bed allocation and bed occupancy in a manner to reduce unwarranted variation; updating rostering of staff required to reduce unwarranted variation; updating times and dates of schedules of activities and processes (such as appointments, procedures, investigations, interventions, etc.) to reduce unwarranted variation; and updating any other system which impacts the activities and processes which could lead to unwarranted variation.

Automatic actions can be initiated by the system if it is deemed that the actions are safe and would reduce activities and processes that would result in unwarranted variation, or initiate activities and processes that would reduce unwarranted variation or improve the chances that the patient follows the desired pathway. Examples of automatic actions include prescribing or cancelling medications or other health interventions, investigations, clinical procedures, or operational procedures. For example, if there is healthcare variation in a pathway, where the desired pathway is antiplatelet therapy, and patients which follow the pathway with unwarranted variations are prescribed anti-thrombin therapy, the system would use automated actions to ensure patients do not follow the pathway with unwarranted variation. This can be performed using conformance checking to identify patients that were following the pathway with unwarranted variation, and being prescribed anti-thrombin medication, and change their prescription to anti-platelet. Alternatively, conformance checking can also be done proactively by identifying that the patient is meant to be following the desired pathway, and proactively prescribing the anti-platelet medication to ensure that the patient follows the desired pathway and not a pathway with unwarranted variation. This operation monitoring will reduce the amount of unwarranted variation and thereby improve patient outcomes and key performance metrics associated with unwarranted variation. Automatic scheduling involves the reordering of tasks, processes, and activities, as well as the allocation of resources (e.g. staff) to activities and processes, in order to reduce unwarranted variation. This can be done via optimisation of the scheduling of activities and processes to minimise or maximise key performance metrics linked to following a path known to have less unwarranted variation. This can automatically restructure hospital workflow to a structure that minimises the number of patients following paths with unwarranted variation. This is because variation can exist in the order and position in a healthcare pathway in which processes and activities occurs, as well as if they occur at all. Two pathways may have the same activities and processes, although the order in which the activities occur in the pathway may showcase unwarranted variation (thereby impacting patient outcome and key performance metrics). For example, given the following pathways:
*Group A (first treatment within 62 days)*
   1^{st} Appointment → Colonoscopy → Histology → CT Scan → MDT Meeting → 1^{st} Treatment
*Group B (first treatment not within 62 days)*
   Colonoscopy → 1^{st} Appointment → Histology → CT Scan → MDT Meeting → 1^{st} Treatment

Variation can be seen in the ordering of the first two activities. Automatic scheduling would re-order the first two activities using operational monitoring and conformance checking so that the activities occurred in the order that reduced unwarranted variation as seen in the pathway for Group A. The schedules of activities and processes can be initiated and communicated to initiate change in real life healthcare pathways in multiple ways e.g. alerts and notifications, automatic updates, automatic activities and actions.

### Improvement cycle

The steps and methods discussed above are implementable in a cyclic manner. The cycle comprises four stages:
(1) Identification of an area of a pathway to focus on (macro analysis);
(2) Identification and assessment of variation (micro analysis);
(3) Understanding the impact of this variation and its impact on patient outcomes and key performance indicators (actionable insight); and
(4) Implementation of actionable change in healthcare pathways, activities, and processes to reduce unwarranted variation (service improvement).

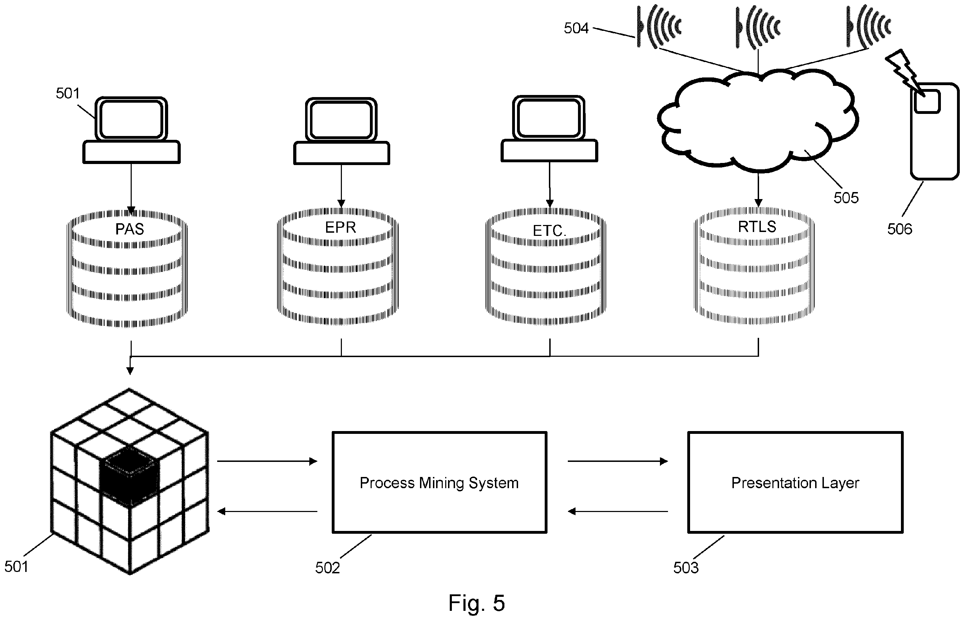

Figure 5 illustrates a system which is operable to perform the methods discussed above. The system uses a number of information sources, data from which are integrated into process cubes 501 as discussed previously. These process cubes are mined by a process mining system 502, and information resulting from this provided to a presentation layer 503 (e.g. a CPV). The information sources include a patient administration system (PAS); electronic patient record (EPR); real-time location system (RTLS); and other information sources (ETC.).

The process mining system 502 comprises one or more processors and memory, the memory contains machine executable instructions which, when run on the processor(s) causes them to acquire healthcare process data from the process cube 501. As discussed previously, this healthcare process data describes event occurring in relation to a plurality of patients in the healthcare system under investigation. The processor(s) then analyse the healthcare process data to identify an actual clinical pathway taken by each patient through the healthcare system, and variations are determined in those pathways. The determined variations are then provided to the presentation layer, in this example a clinical process visualizer, for presentation and interpretation.

The RTLS system utilises a number of sensors 504 connected to a network 505. The sensors detect the movement and/or position of a physical object 506 (e.g. a patient or machine tag). For example, a transmission example from the sensors may include: a time stamp; a location data or identification of the sensor; and the detected physical objects identification. The PAS, EPR and other information sources typically acquire data from a human machine interface 501 where a member of staff enters information to be stored. A transmission example from the PAS, EPR, or other information source may include: a time stamp; a location data or identification of the human machine interface; and identifier information of the user entering the information.

In one example, a first sensor located at the entrance to a ward may sense at a first time stamp that a physical object associated with a patient has passed nearby. The system can infer therefrom that the patient is being moved. Subsequently, a second sensor located at the entrance to a CT scanner room senses at a second time stamp that the same physical object has passed nearby. The system can thereby infer that the patient was near or in the CT scanner room. Shortly thereafter, a CT scan image is registered, by a user, on the system and associated with the patient. The system can infer therefore that the patient was undergoing a CT scan shortly after the second sensor sensed the physical object associated with the patient.

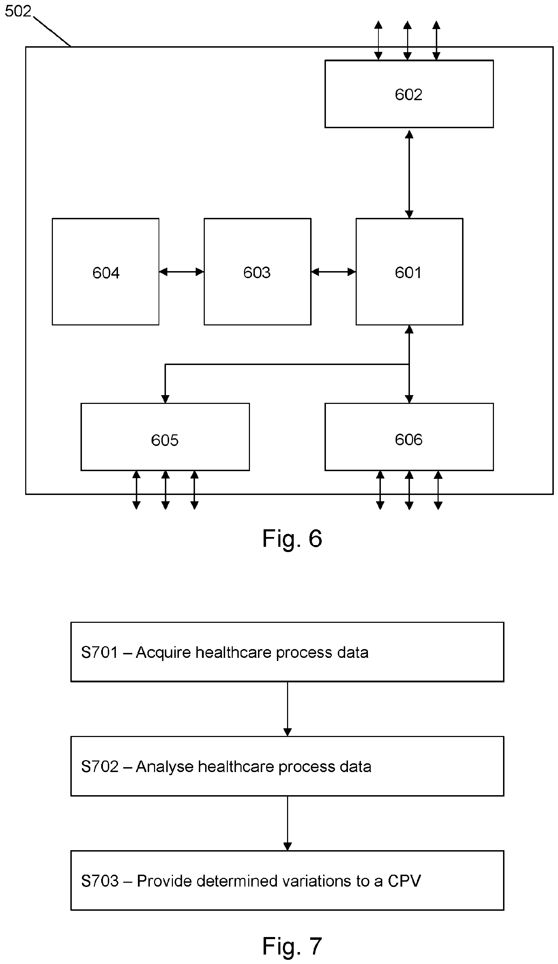

Figure 6 shows the process mining system 502 in more detail. The system comprises a processor 601, which is in communication with a network interface 602. It is with this network interface 602 that the system 502 communicates with external systems for the retrieval of data. The processor is also connected to memory 603, which contain the machine executable instructions referred to above. The memory 603 is in communication with, and retrieves data from, local storage 604 (e.g. a hard drive, or other persistent storage device). The local storage 604 may contain a further copy of the machine executable instructions, which may be loaded into memory 603 when the system is in operation. The processor is also connected to input/output interface 605, which allows for communication with external storage and other peripheral devices (e.g. human machine interface devices). The processor is also connected, via display adapter 606, to a presentation device such as a screen.

Figure 7 shows a flow diagram of a method according to the disclosure. In a first step, S701, healthcare process data is acquired in the manner discussed above. The healthcare process data describes events occurring in relation to a plurality of patients in a healthcare system. The method moves to step S702, where the healthcare process data is analysed in the manner discussed above. The analysis identifies an actual clinical pathway taken by each patient through the healthcare system, and determines variations in those pathways. The method moves to step S703, where the determined variations are provided to a clinical process visualizer, whereupon they are displayed for interpretation.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. Preferably the computer system has a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

## Claims

1. A computer-implemented method for identifying unwarranted healthcare variation in a healthcare system, the method comprising:
(a) acquiring healthcare process data, the healthcare process data describing events occurring in relation to a plurality of patients in the healthcare system;
(b) analysing the healthcare process data to identify an actual clinical pathway taken by each patient through the healthcare system, and to determine variations in those pathways; and
(c) providing the determined variations to a clinical process visualizer, the clinical process visualizer displaying the determined variations for interpretation.

2. The computer-implemented method of claim 1, wherein the method includes varying a healthcare system workflow to be experienced by one or more of the patients based on the determined variations.

3. The computer-implemented method of claim 1 or claim 2, wherein in step (b), variations are determined between the actual clinical pathways for respective patients.

4. The computer-implemented method of any preceding claim, wherein in step (b), variations are determined between the actual clinical pathway for each patient and a reference clinical pathway.

5. The computer-implemented method of any preceding claim, wherein the healthcare process data describes events occurring in relation to a plurality of patients in the healthcare system, wherein the plurality of patients are selected based on an expected clinical pathway.

6. The computer-implemented method of any preceding claim, wherein the healthcare process data describes events occurring in relation to a plurality of patients in the healthcare system, wherein the plurality of patients are selected based on priority rankings of the patients.

7. The computer-implemented method of any preceding claim, wherein the method includes a step, performed before step (a), of receiving one or more key performance metrics associated with the healthcare system, and wherein the data acquired in step (a) at least partially describes the key performance metrics.

8. The computer-implemented method of any preceding claim, wherein the healthcare process data is acquired in the form of process cubes, which contain an event log structure at a plurality of levels of granularity.

9. The computer-implemented method of claim 8, wherein the process cubes integrate data from a plurality of data sources.

10. The computer-implemented method of any preceding claim, wherein acquiring the healthcare process data includes identifying missing or erroneous event data, and providing or correcting the missing or erroneous data by imputation before the healthcare process data is analysed.

11. The computer-implemented method of claim 10, wherein the imputation of the missing or erroneous data uses real time location systems which track one or more physical objects associated with a patient.

12. The computer-implemented method of claim 10 or 11, wherein the imputation of the missing or erroneous data uses data from a plurality of sources, linked by an event occurring in relation to a given patient.

13. The computer-implemented method of any preceding claim, wherein analysing the healthcare process data includes applying a conformance checking algorithm, configured to determine an individual patient's conformance to a reference clinical pathway.

14. The computer-implemented method of claim 13, wherein the reference clinical pathway has been derived by pre-analysing the healthcare process data to determine a clinically desired pathway.

15. The computer-implemented method of any preceding claim, wherein identifying the actual clinical pathway taken by each patient is identified by use of either a hierarchical process mining method, or a declarative process mining method.

16. The computer-implemented method of any preceding claim, wherein the method comprises repeating steps (a) - (c)

17. The computer implemented method of any preceding claim, further including a step of deriving, from the determined variations, an improvement to the healthcare system to reduce unwarranted healthcare variation.

18. The computer-implemented method of claim 17, further including a step of implementing the improvement to the healthcare system.

19. A computer system for identifying unwarranted healthcare variation in a healthcare system, the system comprising one or more processors and memory, the memory containing machine executable instructions which, when run on the one or more processors, causes the one or more processors to:
(a) acquire healthcare process data, the healthcare process data describing events occurring in relation to a plurality of patients in the healthcare system;
(b) analyze the healthcare process data to identify an actual clinical pathway taken by each patient through the healthcare system, and to determine variations in those pathways; and
(c) provide the determined variations to a clinical process visualizer, the clinical process visualizer displaying the determined variations for interpretation.

20. The computer system of claim 19, wherein the memory contains machine executable instructions which, when run on the one or more processors, cause the one or more processors to perform the computer-implemented method of any of claims 1 - 18.

## Amended claims

### Amended claims in accordance with Rule 137(2) EPC.

1. A computer-implemented method for identifying unwarranted healthcare variation in a healthcare system, the method comprising:
(a) acquiring healthcare process data, the healthcare process data describing events occurring in relation to a plurality of patients in the healthcare system;
(b) analysing the healthcare process data to identify an actual clinical pathway taken by each patient through the healthcare system, and to determine variations in those pathways; and
(c) providing the determined variations to a clinical process visualizer, the clinical process visualizer displaying the determined variations for interpretation;
wherein acquiring the healthcare process data includes identifying missing or erroneous event data, and providing or correcting the missing or erroneous data by imputation before the healthcare process data is analysed, wherein imputation of the missing or erroneous data uses real time location systems which track one or more physical objects associated with a patient.

2. The computer-implemented method of claim 1, wherein the method includes varying a healthcare system workflow to be experienced by one or more of the patients based on the determined variations.

3. The computer-implemented method of claim 1 or claim 2, wherein in step (b), variations are determined between the actual clinical pathways for respective patients.

4. The computer-implemented method of any preceding claim, wherein in step (b), variations are determined between the actual clinical pathway for each patient and a reference clinical pathway.

5. The computer-implemented method of any preceding claim, wherein the healthcare process data describes events occurring in relation to a plurality of patients in the healthcare system, wherein the plurality of patients are selected based on an expected clinical pathway.

6. The computer-implemented method of any preceding claim, wherein the healthcare process data describes events occurring in relation to a plurality of patients in the healthcare system, wherein the plurality of patients are selected based on priority rankings of the patients.

7. The computer-implemented method of any preceding claim, wherein the method includes a step, performed before step (a), of receiving one or more key performance metrics associated with the healthcare system, and wherein the data acquired in step (a) at least partially describes the key performance metrics.

8. The computer-implemented method of any preceding claim, wherein the healthcare process data is acquired in the form of process cubes, which contain an event log structure at a plurality of levels of granularity.

9. The computer-implemented method of claim 8, wherein the process cubes integrate data from a plurality of data sources.

10. The computer-implemented method of claim 1, wherein the imputation of the missing or erroneous data uses data from a plurality of sources, linked by an event occurring in relation to a given patient.

11. The computer-implemented method of any preceding claim, wherein analysing the healthcare process data includes applying a conformance checking algorithm, configured to determine an individual patient's conformance to a reference clinical pathway.

12. The computer-implemented method of claim 11, wherein the reference clinical pathway has been derived by pre-analysing the healthcare process data to determine a clinically desired pathway.

13. The computer-implemented method of any preceding claim, wherein identifying the actual clinical pathway taken by each patient is identified by use of either a hierarchical process mining method, or a declarative process mining method.

14. The computer-implemented method of any preceding claim, wherein the method comprises repeating steps (a) - (c).

15. The computer implemented method of any preceding claim, further including a step of deriving, from the determined variations, an improvement to the healthcare system to reduce unwarranted healthcare variation.

16. The computer-implemented method of claim 15, further including a step of implementing the improvement to the healthcare system.

17. A computer system for identifying unwarranted healthcare variation in a healthcare system, the system comprising one or more processors and memory, the memory containing machine executable instructions which, when run on the one or more processors, causes the one or more processors to:
(a) acquire healthcare process data, the healthcare process data describing events occurring in relation to a plurality of patients in the healthcare system;
(b) analyze the healthcare process data to identify an actual clinical pathway taken by each patient through the healthcare system, and to determine variations in those pathways; and
(c) provide the determined variations to a clinical process visualizer, the clinical process visualizer displaying the determined variations for interpretation;
wherein acquiring the healthcare process data includes identifying missing or erroneous event data, and providing or correcting the missing or erroneous data by imputation before the healthcare process data is analysed, wherein imputation of the missing or erroneous data uses real time location systems which track one or more physical objects associated with a patient.

18. The computer system of claim 17, wherein the memory contains machine executable instructions which, when run on the one or more processors, cause the one or more processors to perform the computer-implemented method of any of claims 1 - 16.
